Europäisches Patentamt

European Patent Office

Office européen des brevets

(11) Publication number: **0 344 796 B1**

(12) **EUROPEAN PATENT SPECIFICATION**

(45) Date of publication of patent specification: **21.09.94**  (51) Int. Cl.⁵: **C07K 15/00**, C12P 21/02, //C12N15/27

(21) Application number: **89110022.4**

(22) Date of filing: **02.06.89**

(54) Crystalline human granulocyte colony stimulating factor and process for preparing the same.

(30) Priority: **03.06.88 JP 137111/88**
**14.09.88 JP 231388/88**
**19.05.89 JP 126392/89**

(43) Date of publication of application:
**06.12.89 Bulletin 89/49**

(45) Publication of the grant of the patent:
**21.09.94 Bulletin 94/38**

(84) Designated Contracting States:
**AT BE CH DE ES FR GB IT LI NL SE**

(56) References cited:

**R.K. SCOPES: "Protein Purification. Springer Advanced Texts in Chemistry", 2nd edition, 1987, pages 296-301, 320-321, Springer-Verlag, New York, US**

**D.L. OXENDER et al.: "Protein Engineering", 1986, page 8, Alan R. Liss, Inc., New York, US**

(73) Proprietor: **Chugai Seiyaku Kabushiki Kaisha**
**5-1, 5-chome, Ukima**
**Kita-ku**
**Tokyo (JP)**

(72) Inventor: **Tanaka, Sadao**
**1390-2-301, Kawashimada**
**Gotenba-shi Shizuoka-ken (JP)**
Inventor: **Akimoto, Toshio**
**3002-35, Shimotsuruma**
**Yamato-shi Kanagawa-ken (JP)**

(74) Representative: **VOSSIUS & PARTNER**
**Postfach 86 07 67**
**D-81634 München (DE)**

Rank Xerox (UK) Business Services
(3.10/3.09/3.3.3)

JOURNAL OF BIOLOGICAL CHEMISTRY, vol. 262, no. 25, 5th September 1987, pages 12306-12308, The American Society for Biochemistry and Molecular Biology, Inc., US; B.J. BRANDHUBER et al.: "Crystals and a low resolution structure on interleukin-2"

JOURNAL OF BIOLOGICAL CHEMISTRY, vol. 262, no. 25, 5th September 1987, pages 12323-12324, The American Society for Biochemistry and Molecular Biology, Inc., US; G.L. GILLILAND et al.: "A preliminary crystallographic study of recombinant human interleukin 1beta"

## Description

Human granulocyte colony stimulating factor (hereinafter referred to as human G-CSF) acts upon human bone marrow cells and induces their differentiation and proliferation into granulocytes. Human G-CSF is expected to be applied as a drug for chemotherapy or prevention of various kinds of leukemia and as a therapeutic for serious infectious diseases which cannot be treated with antibiotics alone.

Several types of human G-CSF have been reported. Natural human G-CSF was isolated from the culture supernatant of G-CSF-producing cells which were established from human tumor cells in patients with oral cancer (Japanese Patent Public Disclosure No. 62-227526). G-CSF free from sugar chain was produced by E. coli used as host cells by means of recombinant DNA technology (Japanese Patent Public Disclosure No. 62-132898 and Japanese Patent Public Disclosure No. 62-132899). Glycoprotein G-CSF was also produced by using animal cells as a host (Japanese Patent Public Disclosure No. 62-236497).

Many kinds of techniques have been developed for the crystallization of proteins or polypeptides. In particular, crystallization of enzymes is in fact carried out as being one of the most effective procedures for raising the purity. However, no generalized procedure for crystallization has been developed. The species of enzymes, the conditions of crystallization such as pH, ionic strength, temperature and protein concentration may vary.

Furthermore, some bioactive proteins or polypeptides are very unstable in the face of changes in pH, ionic strength, temperature and so on, which leads to potential changes in three dimensional structure and results in a reduction or total loss of activity. It is therefore necessary to choose appropriate conditions for crystallization which will not lead to any change in three dimesional structure.

It is commonly known that many factors which have to be determined by a great deal of trials and errors are involved in protein crystallization, and thus, in many cases of crystallization, a lot of trials are necessary.

In order to crystallize proteins from an aqueous solution, it is necessary to gradually increase the stronger affinity between protein molecules than between the protein and water. With respect to molecules like proteins having a high molecular weight and having surfaces with complex physical properties, it is of course natural that the law that governs minimization of the free energy of the interaction between proteins holds, but the interaction between proteins is much more complex than that between organic compounds having low molecular weights. It is considered that the various factors which cause interactions between proteins include electronic charges, steric effect, hydrophobic effect, hydrophilic effect and Van-der-Walls effect. The interactions between proteins, and between proteins and water function in such a way that a system containing those proteins is placed in the most stable state. Thus, protein molecules show various types of mutual interaction depending on the concentration, temperature, pH, ionic strength and so on, and in some cases proteins can crystallize while in other cases they cannot crystallize, resulting only in aggregation. Even in a case where protein crystallizes, polymorphism of crystals may be produced depending on such factors as protein concentration, temperature, ionic strength and so on.

It is known that crystallization of proteins of low purity is very difficult to achieve.

As described above, many trials for the crystallization of proteins and polypeptides have been carried out and the crystallization of some proteins has, in fact, been successful. However, most successful cases were limited to just proteins among the great number of naturally occurring proteins and were also limited to very stable proteins such as exocellular proteins and proteins isolated from plants.

The following important advantages are obtained by crystallizing human G-CSF, which has previously thought to have been very difficult. These advantages are:

(1) to heighten the purity and to reduce the antigenicity;

(2) to increase the stability thereby making handling of G-CSF easy during storage and drug preparation; and

(3) to increase the activity of G-CSF.

Fig. 1 is a microscopic photograph (x 100) of crystalline human G-CSF prepared by the method described in Example 1.

Fig. 2 shows the colony formation stimulating activity of crystalline human G-CSF prepared by the method described in Example 1.

Figs. 3a and 3b show HPLC chromatographs of crude human G-CSF and crystalline human G-CSF, respectively [detection, absorbance at 200 nm; elution buffer, 10 mM phosphate buffer (pH 6.8) + 100 mM NaCl; column, GPC (TSK G-3000); flow rate, 1 ml/min.]

Fig. 4 is a precession photograph of (OKℓ) crystals I and II ($\mu$ = 10°) of crystalline human G-CSF prepared by the method of Example 1.

Fig. 5 shows a microscopic photograph (x 84) of crystalline human G-CSF prepared by the method described in Example 5.

Many types of human G-CSF purified by the methods described above (Japanese Patent Public Disclosure Nos. 61-227526, 62-132898, 62-132899 and 62-236497) are dissolved in a neutral or weak acidic buffer and crystallized in the presence of a crystallizing agent, and crystals are then recovered by centrifugation.

It is preferable for crystalline human G-CSF to be purified to such an extent that it contains neither any impurities nor any polymers like dimers or trimers when dissolved in water.

Human G-CSFs which may be employed as starting materials in the instant crystallization process include a natural human G-CSF which is a glycoprotein, a human G-CSF which is produced by animal cells as a host by recombinant DNA technique and its derivatives, and a human G-CSF free from sugar chains which is produced by E. coli as a host by recombinant DNA technique and its derivatives. Generally, crystallization of glycoproteins and proteins having sugar chains is more difficult than that of proteins having no sugar chains, but the instant crystallization procedure makes it possible to crystallize glycoprotein human G-CSFs.

The crystallization of human G-CSF by the instant invention can be carried out by gas-vapor equilibrium methods such as the hanging drop method and the depression slide method; methods using precipitants such as the button method, the dialyzing membrane method and the batch method; and dialysis methods such as the button method and the dialyzing membrane method.

Each of these methods is illustrated below.

Gas-vapor equilibrium method

Representative examples of this method are the hanging drop method and the depression slide method. In both methods, a human G-CSF to be crystallized is dissolved in weak acidic or neutral buffers preferably having a pH of from 3.0 to 7.0 such as acetate buffer, phosphate buffer, citrate buffer, phosphate-citrate buffer with the concentration of from 5 to 100 mM. It is preferred that the concentration of human G-CSF in a buffer is in the range of from about 1 to 100 mg/ml.

A crystallizing agent such as neutral or weak acidic salts e.g. $(NH_4)_2SO_4$, $Na_2SO_4$, NaCl, KCl, $NH_4Cl$, $NaH_2PO_4$, $KH_2PO_4$, $NH_4H_2PO_4$, LiCl, CsCl, $CaCl_2$, $MgCl_2$, $MgSO_4$ and so on and, then, an organic compound e.g. polyethyleneglycol are added to the buffer containing human G-CSF as described above. Although the concentration of the precipitating agent varies depending on the kind of agent and buffer, $(NH_4)_2SO_4$ is used in a concentration of about 1 to 20%, preferably about 2 to 15% of the saturation level. As for other precipitating agents, a concentration of about 0.1 to 1.5 M, preferably about 0.2 to 1.2M is used for crystallization.

Furthermore, if necessary, a stabilizing agent (e.g. neutral salts such as NaCl, KCl, CsCl, $MgCl_2$ and $CaCl_2$ or organic compounds such as glycerol, dioxan and acetone), an antiseptic (e.g. $NaN_3$), a chelating agent for heavy metals (e.g. EDTA), a thiol group protecting agent or an antioxidant (e.g. mercaptoethanol, cysteine, glutathione and dithiothreitol), an antipolymerizing agent, surfactant (e.g. octyl-$\beta$-glycoside, hexantriol, Tweens® or octaethyleneglycol-n-dodecylether) and so on can be added to the protein solution.

In the hanging drop method, droplets of human G-CSF solution prepared by the method described above are put on the back face of a cover glass plate, the glass plate is set on a reservoir which contains a predetermined amount of precipitant and a buffer, usually, the same as that used in preparation of the human G-CSF solution, and the reservoir is sealed completely with silicone grease.

In the depression slide method, a predetermined amount of a buffer and precipitant is placed in a reservoir, human G-CSF solution is put on a depression slide glass and the reservoir is allowed to stand at 5 to 30°C for 1 to 21 days preferably in a quake-proof incubator and crystalline human G-CSF is thus produced.

Salting-out method using dial (Precipitant method)

One to 100 mg/ml human G-CSF solution in a buffer is put in a dialysis tube and is dialyzed against the same buffer containing 10 to 14% saturated precipitating agent [e.g. 10 to 14% saturation of $(NH_4)_2SO_4$, 0.2 to 1.5M NaCl, 0.2 to 1.5M $NaH_2PO_4$ and so on] or polyethyleneglycol solution until crystal grow (usually for 1 week). After that the crystals produced can be isolated by centrifuging the contents in the dialysis tube.

4

Button method and Dialysis method using dialysis membrane

One to 100 mg/ml human G-CSF in a buffer solution is put in a button for crystallization, is sealed with a EDTA-treated dialysis tube and is dialyzed against the same buffer of low concentration for 1 day to 1 week. After that the needle-sphaped crystals produced can be isolated by centrifugation of the contents of the button.

Batch method

One to 100 mg/ml human G-CSF in a buffer solution is put in suitable containers, and a solution of a precipitating agent which is usually dissolved in the same buffer is added to the containers. The resulting solution is stirred as necessary, and the mixture is allowed to stand for about 1 to 2 weeks to produce crystalline human G-CSF as colorless plate crystals.

The crystals can be isolated by centrifugation from the buffer solution in which they are contained.

Characterization of crystals

The crystalline human G-CSF obtained by this invention was analyzed by X-ray diffraction and three kinds of crystals having the following characteristics were identified.

```
I     Crystal form:           orthorhombic
      Space group:            P22₁2₁
      Length of axes (Å):     a ≒ 29, b ≒ 94, c ≒ 111
      Solvent volume (%):     Vs ≒ 37
II    Crystal form:           orthorhombic
      Space group:            P2₁2₁2₁
      Length of axes (Å):     a ≒ 28, b ≒ 100, c ≒ 110
      Solvent volume (%):     Vs ≒ 37
III   Crystal form:           orthorhombic
      Space group:            P22₁2₁
      Length of axes (Å):     a ≒ 29, b ≒ 107, c ≒ 114
      Solvent volume (%):     Vs ≒ 46
```

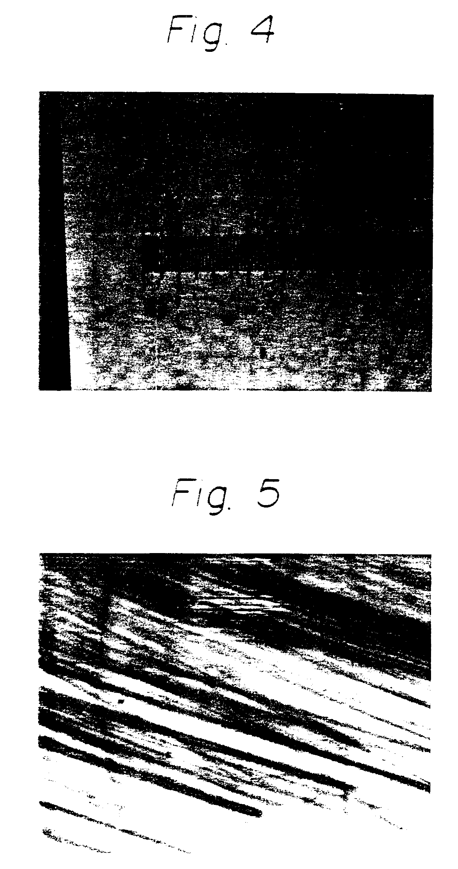

Precession photograph of (OKℓ) of crystals I and II ($\mu$ - 10°) is given as Fig. 4.

In some cases chimeracrystals of the two crystalline substances I and II described above are produced.

Measurement of activity of crystalline human G-CSF

In order to measure the activity of the crystalline human G-CSF, colony stimulating activity (CSA) was assayed in vitro by the following method.

(Assay method)

Assay for CSA was carried out by the soft agar mono-layer cultivation method as in Bradley T. R. and Metealf D. et al. (Aust. J. Exp. Biol, Med. Sci., 44, 287 - 300, 1966). That is, a mixture of 1 ml of horse serum (Hyclone Co., Ltd.), 0.25 ml of test sample, 0.25 ml of C57BL mouse myeloid cell suspension (1.5 x $10^6$ cells/ml) and 1 ml of modified McCoy's 5A medium containing 0.75% agar was poured into a plastic dish for tissure culture. After gelation, the dish was incubated at 37°C or 5°C at 100% R.H. in an atmosphere of 5% $CO_2$/95% air for 5 days, and the number of colonies formed was counted (one colony comprises more than 50 cells). One unit of CSA was defined as being the activity required to form one colony.

A test sample (original solution) was prepared as follows: 10μl of a crystalline human G-CSF solution (14 mg/ml, quantitively analyzed by HPLC) in 100 mM acetate buffer (pH 4.2) was diluted with phosphate buffered saline containing 0.5% mouse serum albumin (Cappel Co., Ltd.) (0.5% mSAPBS) to adjust the concentration to 100 μg/ml (total volume was 1.4 ml). After filtration using a Millipore filter, respective samples of this original solution are diluted 10, 100, 500, 2,500, 12,500 and 62,500 times, respectively, with 0.5% mSAPBS. Also 0.5% mSAPBS was used as a comparison and 1 μg/ml of rG-CSF (noncrystalline, purified human G-CSF produced by recombinant DNA technique was prepared for comparative purposes.

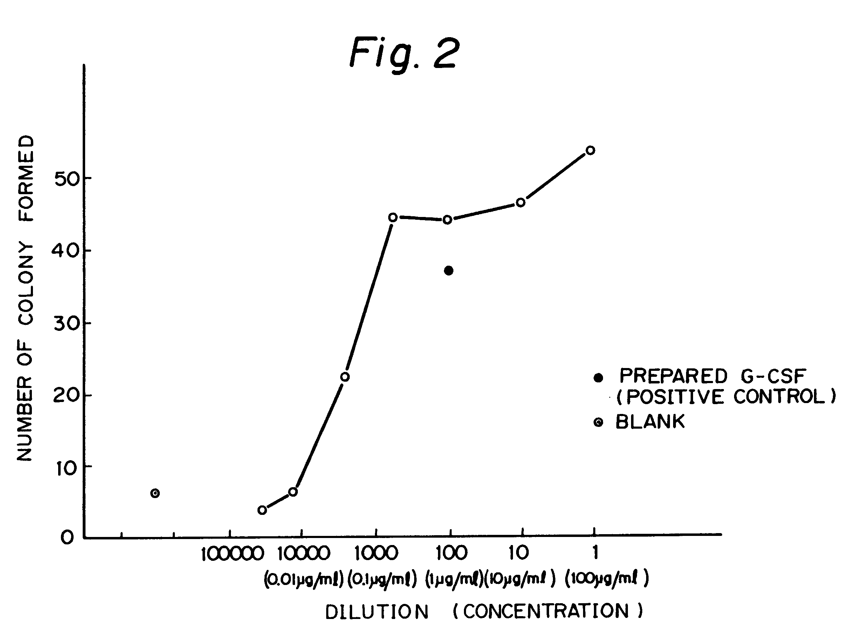

The results are as follows (also shown in Fig. 2):

| Dilution | The number of colonies formed | | |
|---|---|---|---|
| | Experiment 1 | Experiment 2 | Average |
| 1 | 51 | 55 | 54 |
| 10 | 46 | 47 | 46.5 |
| 100 | 44 | X | 44 |
| 500 | 30 | 59 | 44.5 |
| 2,500 | 18 | 27 | 22.5 |
| 12,500 | 6 | 7 | 6.5 |
| 62,500 | 5 | 3 | 4 |
| rG-CSF | 29 | 44 | 36.5 |
| Control | 5 | 7 | 6 |

As clearly shown from the above data, the crystalline human G-CSF of this invention exhibited the same as or higher colony stimulating activity than that of noncrystalline and human G-CSF produced by recombinant DNA technique.

A detailed explanation of this invention is set forth below in regard to Examples. By a similar method, another human G-CSF which exhibits a high degree of sequence homology can be crystallized.

Example 1

Depression slide method (gas-vapor equilibrium method)

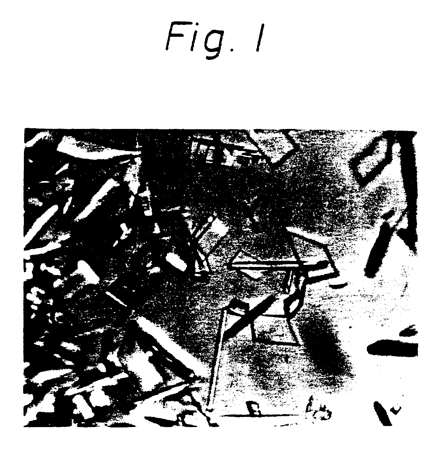

A solution of glycoprotein human G-CSF (10 μl, 15.5 mg/ml), which had been produced by animal cells as a host by recombinant DNA technique, was dissolved in 10 mM citrate buffer (pH 4.6) and the solution was mixed with 16% saturation of a $(NH_4)_2SO_4$ solution (10 μl) on a depression slide glass. One ml of 12% saturation of $(NH_4)_2SO_4$ solution in 10 mM citrate buffer (pH 4.6) was put in a reservoir and the reservoir was covered with a glass plate and sealed with silicone. After the reservoir had been allowed to stand for about 3 weeks at room temperature (20 to 24°C), single crystals of human G-CSF in the form of a colorless, transparent plate were obtained (Fig. 1).

In order to determine the characteristics of the crystals, the signal crystals so produced and the mother liquid were put and sealed in a quartz capillary tube and were analyzed by X-ray diffraction.

The results of the analyses allowed three kinds of crystals having the following characteristics to be identified.

I      Crystal form:           orthorhombic

Space group:         $P22_1 2_1$

Dimension of unit crystal lattice (Å):

a = 29, b = 94, c = 111

Number of molecules per unit crystal lattice:   8

Volume of unit crystal lattice ($Å^3$):   303,900

Solvent volume (%):   Vs = 37

II      Crystal form:           orthorhombic

Space group:         $P2_1 2_1 2_1$

Dimension of unit crystal lattice (Å):

a = 28, b = 100, c = 110

Number of molecules per unit crystal lattice:   8

Volume of unit crystal lattice ($Å^3$):   303,600

Solvent volume (%):   Vs = 37

IlI     Crystal form:           orthorhombic

Space group:         $P22_1 2_1$

Dimension of unit crystal lattice (Å):

a = 29, b = 107, c = 114

Number of molecules per unit crystal lattice:   8

Volume of unit crystal lattice ($Å^3$):   353,742

Solvent volume (%):   Vs = 46

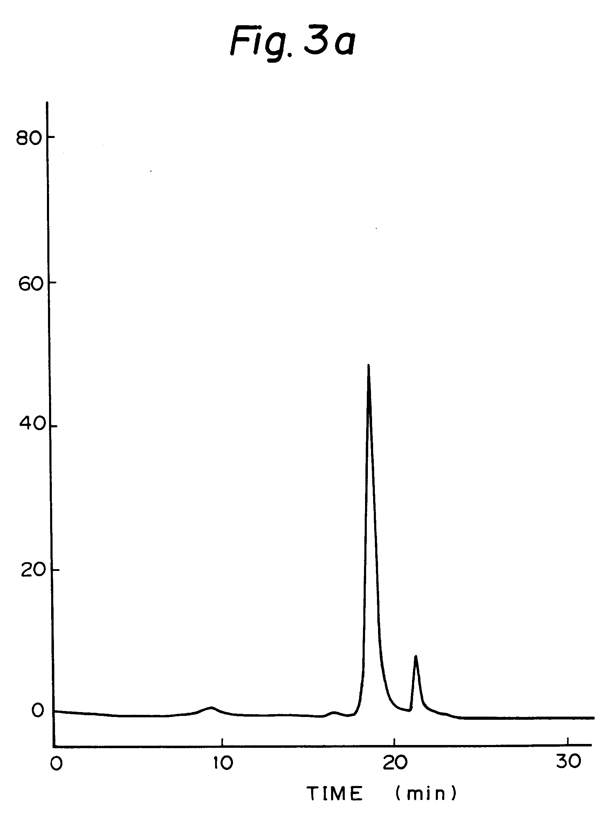

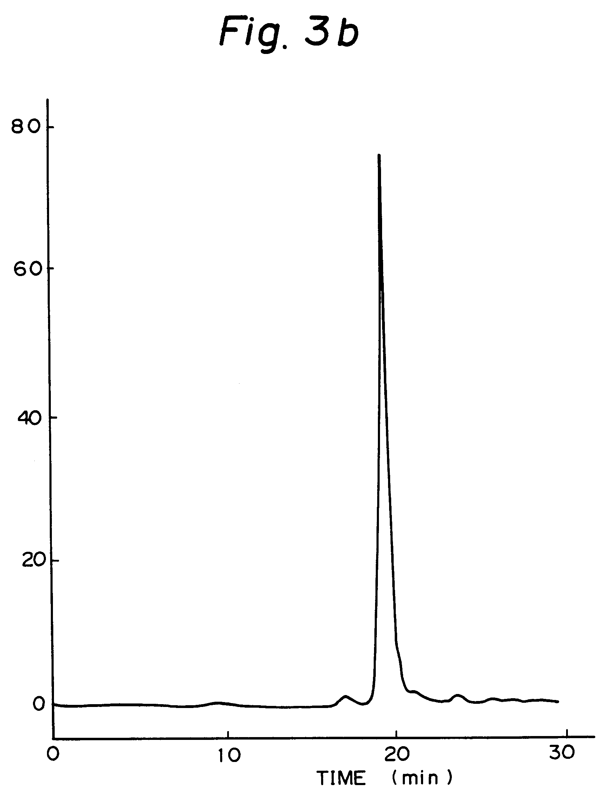

The produced crystal were washed twice with the buffer containing 25% saturation of $(NH_4)_2SO_4$ and then analyzed by HPLC [column, GPC (TSK G-3000); elution buffer, 10 mM phosphate buffer (pH 6.8) + 100 mM NaCl; flow rate, 1 ml/min.] with a retention time of 19.75 minutes. The results of HPLC analyses are shown in Fig. 3a (control) and 3b, which demonstrate that these crystals were G-CSF.

Example 2

Hanging drop method (gas-vapor equilibrium method)

Solutions of the glycoprotein human G-CSF (2 $\mu$l, 18.3 mg/ml) used in Example 1 dissolved in 100 mM acetate buffer (pH 4.2) in which a precipitating agent and/or a stabilizing agent had been dissolved were mixed with various concentrations of $NaH_2PO_4$ solution (2 $\mu$l, 0.8, 0.9, 1.0, 1.1 and 1.2 M) on silanized cover glasses. Reservoirs each containing 8 ml of various concentrations of $NaH_2PO_4$ solutions (0.7, 0.8, 0.9, 1.0, 1.1 and 1.2 M) were covered with the cover glasses, sealed with silicone grease and allowed to stand. Among the various combinations of these samples, colorless plate crystals separated out in about 24 hours in the earliest case.

The crystal which separated out in about 2 weeks was identified as G-CSF by means of HPLC.

The conditions for crystallization of each sample are summarized in the table below.

| Buffer | Crystallizing agent | Reservoir | Stabilizing agent and others |
|---|---|---|---|
| 100 mM acetate buffer (pH 4.2) | 5 - 15% saturated (sat.) $(NH_4)_2SO_2$ (AS) | 10 - 20% sat. AS | none |
| the same as above | 8 - 9% sat. AS 0.4 - 0.8% CsCl | 10 - 20% sat.AS | none |
| the same as above | 5 - 15% sat. AS | 10 - 20% sat. AS (5% glycerol) | glycerol |
| the same as above | 0.3 - 0.5 M NaCl | 0.5 - 1.0 M NaCl | none |
| 10 mM citrate buffer (pH 4.2) | 8 - 9% sat. AS | 10 - 15% sat. AS | none |
| 10 mM citrate/phosphate buffer (pH 5.4) | 8.0 - 12.0% sat. AS | 10 - 18% sat. AS | none |
| 10 mM citrate/phosphate buffer (pH 6.4) | 8.0 - 11.5% sat. AS | 10 - 14% sat. AS | none |
| 100 mM citrate buffer (pH 3.0) | 6.0 - 10% sat. As | 10 - 14% sat. As | none |
| 100 mM phosphate buffer (pH 6.8) | 0.6 - 0.8 M $NaH_2PO_4$ | 0.7 - 1.0 M $NaH_2PO_4$ | 100 mM NaCl |
| 100 mM acetate buffer (pH 4.2) | 8.5 - 12% polyethylene-glycol 4000 | 10 - 15% poly-ethyleneglycol 4000 | 100 mM MgCl2 10 mM CaCl2 0.01% NaN3 1 mM EDTA |
| the same as above | 8.5 - 12% polyethylene-glycol 6000 | 10 - 15% poly-ethyleneglycol 6000 | 100 mM MgCl2 0.5 mM CaCl2 0.01% NaN3 |

### Example 3

Dialysis membrane method

A solution of human G-CSF as used in Example 1 dissolved in 100 mM acetate buffer (pH 4.2) (1050 $\mu$l, 16.3 mg/ml) was sealed in a dialysis tube (pore size: MW 7,000 cut-off, EDTA-pretreated). This tube was soaked in 200 ml of 1 M KCl containing 10 mM acetate buffer (pH 4.2) and was subjected to salting-out for about 1 week with outer solvent being stirred gently. The contents were then transferred to a V-shaped vial and colorless plate crystals were recovered by centrifugation (5°C, 15,000 rpm, 10 minutes).

### Example 4

Batch method

(1) Human G-CSF as used in Example 1 dissolved in 100 mM acetate buffer (pH 4.2) (200 $\mu$l, 15.58 mg/ml) was put in a container. To this solution was added a 100 mM acetate buffer containing 1.2 M $NaH_2PO_4$ and the mixture was allowed to stand for 2 weeks at room temperature.

Colorless plate crystals separated out and were then isolated by centrifugation (5°C, 15,000 rpm, 10 minutes).

(2) As described in (1), a human G-CSF solution in 100 mM acetate buffer (pH 4.2) (300 $\mu$l, 13.08 mg/ml) was mixed with 300 $\mu$l of a 100 mM acetate buffer containing 16% saturation of $(NH_4)_2SO_4$ and the mixture was allowed to stand for 3 weeks. Colorless plate crystals were isolated by centrifugation.

### Example 5

Dialysis membrane method

Human G-CSF as used in Example 1 which had been dialyzed against a 15% saturation of $(NH_4)_2SO_4$ solution was dissolved in 5 mM citrate buffer (pH 4.6). The human G-CSF solution (200 $\mu$l) was put in a dialysis tube and was dialyzed against 80 ml of water. As shown in Fig. 4, very long needle crystals were produced.

### Example 6

Hanging drop method (gas-vapor equilibrium method)

Human G-CSF as used in Example 1 which had been dialyzed against a 15% saturation of $(NH_4)_2SO_4$ was dissolved in a 5 mM citrate buffer containing 3 mM octyl-$\beta$-glycoside (pH 4.6). Five ml of this human G-CSF solution was mixed with 5 ml of a 15 to 22% saturation of $(NH_4)_2SO_4$ solution or with 5 ml of a 5 mM sodium citrate solution. The human G-CSF in the solution was crystallized with use of 8 ml of a 11 to 13% saturation of $(NH_4)_2SO_4$ or a 5 mM sodium citrate solution as an outer solution. Plate crystals were produced in several days to 2 weeks.

### Example 7

Dialysis membrane method

A solution of human G-CSF as used in Example 1 in a 10 mM acetate buffer (pH 4.2) (610 $\mu$l, 11.45 mg/ml) was placed in dialysis tube and then dialyzed against 200 ml of 1 M KCl solution containing a 10 mM acetate buffer (pH 4.2). The KCl concentration of the outer solution was increased from the next day at a rate of 0.1 M per day and finally reached 1.4 M. The contents in dialysis tube were then transferred to a V-shaped vial and centrifuged (10°C, 15,000 rpm, 5 minutes) to remove the liquid phase. Colorless plate crystals were isolated. These crystals were dissolved in 400 $\mu$l of a 10 mM acetate buffer (pH 4.2) and centrifuged (10°C, 12,000 rpm, 5 minutes). The concentration of G-CSF in the resulting supernatant (420 $\mu$l) was determined to be 15.05 mg/ml (yield 90.7%) by HPLC analysis.

9

### Example 8

#### Dialysis membrane method

A solution of human G-CSF as used in Example 1 in a 10 mM acetate buffer (pH 4.2) (680 $\mu$l, 32.09 mg/ml) was poured in dialysis tube and then dialyzed against a 10 mM acetate buffer containing 5% saturation of $(NH_4)_2SO_4$ (200 $\mu$l). The $(NH_4)_2SO_4$ concentration of outer solution was gradually increased from the next day at a rate of 1% per day and finally reached 13%. The contents of the tube were transferred to a V-shaped vial and then centrifuged (10°C, 15,000 rpm, 5 minutes) to remove the liquid phase whereby colorless plate crystals were obtained. The crystals were dissolved in 1,200 $\mu$l of a 10 mM acetate buffer (pH 4.2) and the solution was centrifuged (10°C, 12,000 rpm, 5 minutes). The concentration of G-CSF in the resulting supernatant (1290 $\mu$l) was determined to be 16.32 mg/ml (yield 96.4%) by HPLC analysis.

### Example 9

#### Batch method

To a solution of human G-CSF as used in Example 1 in a 10 mM acetate buffer (pH 4.2) (200 $\mu$l, 19.84 mg/ml) was added a 1.5 M $NaH_2PO_4$ solution (250 $\mu$l) and the mixture was allowed to stand for 1 week. The contents were then transferred to a V-shaped vial and colorless plate crystals were isolated by centrifugation (10°C, 15,000 rpm, 10 minutes). The collected crystals were dissolved in 400 $\mu$l of a 10 mM acetate buffer and the solution was centrifuged (10°C, 12,000 rpm, 10 minutes). The concentration of G-CSF in the resulting supernatant (450 $\mu$l) was determined to be 12.51 mg/ml (yield 78.8%) by HPLC analysis.

### Example 10

#### Batch method

To a solution of human G-CSF as used in Example 1 in a 10 mM acetate buffer (pH 4.2) (1,200 $\mu$l, 28.91 mg/ml) on ice was added a 34% saturation of $(NH_4)_2SO_4$ solution (800 $\mu$l). The mixture was allowed to stand at 14°C and the temperature was gradually increased to 24°C at a rate of 1°C per day. The contents were transferred to a V-shaped vial and colorless plate crystals were isolated by centrifugation (10°C, 15,000 rpm, 5 minutes) to remove the liquid phase. The crystals were dissolved in a 10 mM acetate buffer (pH 4.2) and the solution was centrifuged (10°C, 12,000 rpm, 5 minutes). The concentration of G-CSF in the resulting supernatant (1380 $\mu$l) was determined by HPLC and found to be 24.05 mg/ml (yield 95.7%).

Crystalline human G-CSF prepared by the instant invention was successfully obtained in a high yield (78.8 to 96.4%) as shown in Example 7 to 10. Furthermore, as shown in Fig. 2, crystalline human G-CSF prepared according to this invention exhibited colony stimulating activity that was the same as or higher than that of noncrystalline human G-CSF. From the view point of this activity, this invention is very useful for applications in the medical and pharmaceutical fields. The human G-CSF of this invention also offers great advantages in allowing of easy formulation into for dosage forms because it is in a crystalline form and is very stable.

### Example 11

#### Button method

Human G-CSF obtained by the salting-out method was dissolved in a 5 mM citrate buffer (pH 4.6) to give a final concentration of about 10 mg/ml. The solution was put in a 30 $\mu$l capacity button for crystallization and sealed with EDTA-treated dialysis membrane, carefully avoiding the inclusion of bubbles in it. The membrane was fixed firmly with a rubber ring. This button was dialyzed against a 2 mM citrate buffer to give needle crystals in 1 - 3 days.

Under the following conditions, crystalline human G-CSF was obtained by the same method.

| Buffer | | Detergent | Crystal shape |
|---|---|---|---|
| pH | Concentration (solvent → outer solution) | | |
| Hepes buffer (pH 5.6) | 20 mM → 3 mM | - | micro |
| same as above | 20 mM → 3 mM<br>20 mM → 5 mM | 1 mM octyl-$\beta$-glycoside | plate |
| citrate buffer (pH 4.6) | 10 mM → 3 mM<br>10 mM → 5 mM | - | needle |
| same as above | 5 mM → 3 mM<br>5 mM → 2 mM<br>5 mM → 1 mM | - | needle |
| same as above | 10 mM → 5 mM<br>10 mM → 3 mM | 1 mM octa-ethylene glycol-n-dodesylether | needle |
| same as above | 10 mM → 5 mM<br>10 mM → 3 mM | 1 mM octyl-$\beta$-glycoside | needle |
| same as above | 5 mM → 3 mM<br>5 mM → 2 mM<br>5 mM → 1 mM | same as above | needle |

## Claims

1. Human granulocyte colony stimulating factor in crystalline form.

2. Human granulocyte colony stimulating factor in crystalline form according to Claim 1 wherein the human granulocyte colony stimulating factor is produced by animal cells or other eukaryotic cells by recombinant DNA technique.

3. Human granulocyte colony stimulating factor in crystalline form according to Claim 1 wherein the human granulocyte colony stimulating factor is isolated from culture media of human cells which have the ability to produce the granulocyte colony stimulating factor.

4. Human granulocyte colony stimulating factor in crystalline form according to Claim 1 wherein the human granulocyte colony stimulating factor is produced by a prokaryotic microorganisms as a host by recombinant DNA technique.

5. A process for preparing crystalline human granulocyte colony stimulating factor which comprises dissolving a human granulocyte colony stimulating factor and optionally a precipitating agent in a buffer containing 5 to 100 mM of a buffering salt and having a pH of from 3.0 to 7.0, and thereafter crystallizing the granulocyte colony stimulating factor by the gas-vapor equilibrium method, dialysis method, button method or batch method.

6. A process for preparing human granulocyte colony stimulating factor in crystalline form according to Claim 5 wherein human granulocyte colony stimulating factor is dissolved in a buffer at a concentration of about 1 to 100 mg/ml.

7. A process for preparing human granulocyte colony stimulating factor in crystalline form according to Claim 5 wherein the precipitating agent, when used, is selected from the group consisting of $(NH_4)_2SO_4$, $Na_2SO_4$, $NaCl$, $KCl$, $NH_4Cl$, $NaH_2PO_4$, $KH_2PO_4$, $NH_4H_2PO_4$, $LiCl$, $CsCl$, $CaCl_2$, $MgCl_2$, $MgSO_4$, polyethyleneglycol, sodium citrate, sodium oxalate and mixtures thereof.

8. A process for preparing a human granulocyte colony stimulating factor in crystalline form according to Claim 7 wherein the concentration of the precipitating agent in the solution containing the human granulocyte colony stimulating factor ranges from 0.1 to 1.5 M.

**Patentansprüche**

1. Menschlicher Granulocyten-koloniestimulierender Faktor in kristalliner Form.

2. Menschlicher Granulocyten-koloniestimulierender Faktor in kristalliner Form nach Anspruch 1, wobei der menschliche Granulocyten-koloniestimulierende Faktor von tierischen oder anderen eukaryotischen Zellen durch rekombinante DNA-Technik hergestellt wird.

3. Menschlicher Granulocyten-koloniestimulierender Faktor in kristalliner Form nach Anspruch 1, wobei der menschliche Granulocyten-koloniestimulierende Faktor aus dem Kulturüberstand menschlicher Zellen isoliert wird, die die Fähigkeit haben, den Granulocyten-koloniestimulierenden Faktor herzustellen.

4. Menschlicher Granulocyten-koloniestimulierender Faktor in kristalliner Form nach Anspruch 1, wobei der menschliche Granulocyten-koloniestimulierende Faktor von einem prokariotischen Mikroorganismus als Wirt durch rekombinante DNA-Technik hergestellt wird.

5. Verfahren zur Herstellung von kristallinem menschlichem Granulocyten-koloniestimulierendem Faktor, wobei man einen menschlichen Granulocyten-koloniestimulierenden Faktor und gegebenenfalls ein Fällungsmittel in einem Puffer enthaltend 5 bis 100 mM eines Puffersalzes und mit einem pH-Wert von 3,0 bis 7,0 löst und sodann den Granulocyten-koloniestimulierenden Faktor mit dem Gas-Dampf-Gleichgewichtsverfahren, Dialyse-Verfahren, Knopfverfahren (Button method) oder Batchverfahren auskristallisiert.

6. Verfahren zur Herstellung von menschlichem Granulocytenkoloniestimulierendem Faktor in kristalliner Form nach Anspruch 5, wobei der menschliche Granulocyten-koloniestimulierende Faktor in einem Puffer bei einer Konzentration von etwa 1 bis 100 mg/ml gelöst wird.

7. Verfahren zur Herstellung von menschlichem Granulocytenkoloniestimulierendem Faktor in kristalliner Form nach Anspruch 5, wobei das Fällungsmittel, sofern verwendet, ausgewählt wird aus der Gruppe $(NH_4)_2SO_4$, $Na_2SO_4$, $NaCl$, $KCl$, $NH_4Cl$, $NaH_2PO_4$, $KH_2PO_4$, $NH_4H_2PO_4$, $LiCl$, $CsCl$, $CaCl_2$, $MgCl_2$, $MgSO_4$, Polyethylenglycol, Natriumcitrat, Natriumoxalat und Gemischen davon.

8. Verfahren zur Herstellung eines menschlichen Granulocyten-koloniestimulierenden Faktors in kristalliner Form nach Anspruch 7, wobei die Konzentration des Fällungsmittels in der Lösung enthaltend den menschlichen Granulocyten-koloniestimulierenden Faktor in einem Bereich von 0,1 bis 1,5 M liegt.

**Revendications**

1. Facteur humain de stimulation de colonies de granulocytes sous forme cristalline.

2. Facteur humain de stimulation de colonies de granulocytes sous forme cristalline suivant la revendication 1, dans lequel le facteur humain de stimulation de colonies de granulocytes est produit par des cellules animales ou autres cellules eucaryotes par la technique de l'ADN recombiné.

3. Facteur humain de stimulation de colonies de granulocytes sous forme cristalline suivant la revendication 1, dans lequel le facteur humain de stimulation de colonies de granulocytes est isolé d'un milieu de culture de cellules humaines qui ont la capacité de produire le facteur de stimulation de colonies de granulocytes.

4. Facteur humain de stimulation de colonies de granulocytes sous forme cristalline suivant la revendication 1, dans lequel le facteur humain de stimulation de colonies de granulocytes est produit par des micro-organismes procaryotes en tant qu'hôte, par la technique de l'ADN recombiné.

5. Procédé de préparation d'un facteur cristallin humain de stimulation de colonies de granulocytes qui comprend la dissolution d'un facteur humain de stimulation de colonies de granulocytes et facultativement d'un agent de précipitation dans un tampon contenant 5 à 100 mM d'un sel de tamponnage et ayant un pH de 3,0 à 7,0 et ensuite, cristallisation du facteur de stimulation de colonies de granulocytes par le procédé d'équilibre gaz-vapeur, un procédé de dialyse, un procédé de culot ou un procédé discontinu.

6. Procédé de préparation d'un facteur humain de stimulation de colonies de granulocytes sous forme cristalline suivant la revendication 5, dans lequel le facteur humain de stimulation de colonies de granulocytes est dissous dans un tampon à une concentration d'environ 1 à 100 mg par ml.

7. Procédé de préparation d'un facteur humain de stimulation de colonies de granulocytes sous forme cristalline suivant la revendication 5, dans lequel l'agent de précipitation, lorsqu'il est utilisé, est choisi parmi le groupe consistant en $(NH_4)_2SO_4$, $Na_2SO_4$, $NaCl$, $KCl$, $NH_4Cl$, $NaH_2PO_4$, $KH_2PO_4$, $NH_4H_2PO_4$, $LiCl$, $CsCl$, $CaCl_2$, $MgCl_2$, $MgSO_4$, polyéthylèneglycol, citrate de sodium, oxalate de sodium et des mélanges de ceux-ci.

8. Procédé de préparation d'un facteur humain de stimulation de colonies de granulocytes sous forme cristalline suivant la revendication 7, dans lequel la concentration de l'agent de précipitation dans la solution contenant le facteur humain de stimulation de colonies de granulocytes se situe entre 0,1 et 1,5 M.

# Fig. 1

Fig. 2

# Fig. 3a

# Fig. 3b

## Fig. 4

## Fig. 5